# EUROPÄISCHE PATENTSCHRIFT

(11) **EP 3 060 112 B1**
(45) Veröffentlichungstag und Bekanntmachung des Hinweises auf die Patenterteilung: **20.11.2019**
(21) Anmeldenummer: 14801909.4
(22) Anmeldetag: 25.09.2014
(51) Int. Cl.: A61B 5/0484, A61B 5/16

(54) **VERFAHREN ZUR QUANTIFIZIERUNG DER WAHRNEHMUNGSFÄHIGKEIT EINER PERSON**
METHOD FOR QUANTIFYING THE PERCEPTIVE FACULTY OF A PERSON
PROCÉDÉ DE QUANTIFICATION DE LA CAPACITÉ DE PERCEPTION D'UNE PERSONNE

(30) Priorität: 21.10.2013 AT 506762013
(43) Veröffentlichungstag der Anmeldung: 31.08.2016
(73) Patentinhaber: Guger, Christoph, 4533 Piberbach (AT); Edlinger, Günter, 8020 Graz (AT)
(72) Erfinder: Guger, Christoph, 4533 Piberbach (AT); Edlinger, Günter, 8020 Graz (AT)
(74) Vertreter: Wildhack & Jellinek
(86) Internationale Anmeldenummer: PCT/AT2014/050218
(87) Internationale Veröffentlichungsnummer: WO 2015/058223

(56) Entgegenhaltungen:
- US-A1- 2005 017 870
- US-A1- 2009 062 679
- VLEK R J ET AL: "Sequenced subjective accents for braincomputer interfaces;Sequenced subjective accents for brain-computer interfaces", JOURNAL OF NEURAL ENGINEERING, INSTITUTE OF PHYSICS PUBLISHING, BRISTOL, GB, Bd. 8, Nr. 3, 4. April 2011 (2011-04-04), Seite 36002, XP020205962, ISSN: 1741-2552, DOI: 10.1088/1741-2560/8/3/036002
- F. BERNASCONI ET AL: "Noise in Brain Activity Engenders Perception and Influences Discrimination Sensitivity", JOURNAL OF NEUROSCIENCE, Bd. 31, Nr. 49, 7. Dezember 2011 (2011-12-07), Seiten 17971-17981, XP055159341, ISSN: 0270-6474, DOI: 10.1523/JNEUROSCI.3715-11.2011
- JAN-NIKLAS ANTONS ET AL: "Analyzing Speech Quality Perception Using Electroencephalography", IEEE JOURNAL OF SELECTED TOPICS IN SIGNAL PROCESSING, IEEE, US, Bd. 6, Nr. 6, 1. Oktober 2012 (2012-10-01) , Seiten 721-731, XP011460707, ISSN: 1932-4553, DOI: 10.1109/JSTSP.2012.2191936
- GUGER C ET AL: "Real-Time EEG Analysis with Subject-Specific Spatial Patterns for a Brain-Computer Interface (BCI)", IEEE TRANSACTIONS ON REHABILITATION ENGINEERING, IEEE INC. NEW YORK, US, Bd. 8, Nr. 4, 1. Dezember 2000 (2000-12-01), XP011053741, ISSN: 1063-6528
- T. K. GUTTORM ET AL: "Event-related potentials in newborns with and without familial risk for dyslexia: principal component analysis reveals differences between the groups", JOURNAL OF NEURAL TRANSMISSION, Bd. 110, Nr. 9, 1. September 2003 (2003-09-01), Seiten 1059-1074, XP055159289, ISSN: 0300-9564, DOI: 10.1007/s00702-003-0014-x
- STEPHEN HUFF J ET AL: "Emergency Neurological Life Support: Approach to the Patient with Coma", NEUROCRITICAL CARE, HUMANA PRESS INC, NEW YORK, vol. 17, no. 1, 30 August 2012 (2012-08-30), pages 54-59, XP035114475, ISSN: 1556-0961, DOI: 10.1007/S12028-012-9755-4
- COLON E ET AL: "Steady-state evoked potentials to study the processing of tactile and nociceptive somatosensory input in the human brain", NEUROPHYSIOLOGIE CLINIQUE - CLINICAL NEUROPHYSIOLOGY, vol. 42, no. 5, 22 June 2012 (2012-06-22), pages 315-323, XP028944018, ISSN: 0987-7053, DOI: 10.1016/J.NEUCLI.2012.05.005

## Beschreibung

Die Erfindung betrifft ein Verfahren zur Quantifizierung der Wahrnehmungsfähigkeit einer Person gemäß dem Patentanspruch 1.

Aus dem Stand der Technik sind unterschiedliche Messverfahren bekannt, die dazu eingesetzt werden können, unterschiedliche gedankliche Tätigkeiten einer Person zu detektieren. Aus dem Stand der Technik sind auch einzelne, sogenannte Gehirn-Computer-Schnittstellen, engl. brain computer interfaces, bekannt, mit denen auf unterschiedliche Weise die im Inneren des Gehirns einer Person ablaufenden Vorgänge ermittelt, weiterverarbeitet und auch visualisiert werden können.

Solche Schnittstellen sind von erheblicher Bedeutung, wenn der betreffenden Person keinerlei sonstige Möglichkeiten zur Kommunikation wie Sprache, Gebärden usw. zur Verfügung stehen. Wesentlicher Hintergrund der Erfindung ist es, Patienten im Spätstadium von neurologischen Erkrankungen, beispielsweise im Spätstadium der amyotrophen Lateralsklerose (ALS), oder auch Patienten mit Bewußtseinsstörungen daraufhin zu untersuchen, wie groß deren individuelle subjektive Wahrnehmungsfähigkeit zu einem bestimmten Zeitpunkt ist. Die Bestimmung dieser Fähigkeiten ist für den betreffenden Patienten von größter Relevanz, da abhängig von der persönlichen Wahrnehmungsfähigkeit an ihn angepasste Kommunikationsmöglichkeiten verwendet werden können.

Durch eine Quantifizierung der Wahrnehmungsfähigkeit kann ermittelt werden, inwieweit diese Person, im folgenden als Proband bezeichnet, zur Kommunikation mit ihrer Umwelt noch in der Lage ist. Vorteilhaft ist es darüber hinaus, wenn die bei der Quantifizierung der Wahrnehmungsfähigkeit notwendigerweise erworbenen Lernerfolge durch den Probanden auch für eine nachfolgenden computergestützte Kommunikation genutzt werden können.

Aus STEPHEN HUFF J ET AL: "Emergency Neurological Life Support: Approach to the Patient with Coma",NEUROCRITICAL CARE, HUMANA PRESS INC, NEW YORK, Bd. 17, Nr. 1, 30. August 2012, Seiten 54-59 ist ein Verfahren zur Feststellung der Wahrnehmungsfähigkeit einer Person bekannt.

Aus dem Stand der Technik sind Verfahren bekannt, die ausschließlich ausgehend von bereits vorhandenen Messwerten Klassifikationen von EEG-Daten ermitteln können. Solche Verfahren sind mitunter jedoch sehr wenig konklusiv, da bei EEG-Daten durchaus individuelle Unterschiede zwischen einzelnen Probanden bestehen.

Die vorliegende Erfindung setzt sich zur Aufgabe, ein rasches und einfaches Verfahren zur Quantifizierung der Wahrnehmungsfähigkeit zur Verfügung zu stellen, das eine wirksame Feststellung der Wahrnehmungsfähigkeit ermöglicht, und von individuellen Unterschieden zwischen einzelnen Probanden weitestgehend unabhängig ist.

Die Erfindung löst die Aufgabe mit den Merkmalen des Patentanspruchs 1.

Erfindungsgemäß ist bei einem Verfahren zur Quantifizierung der Wahrnehmungsfähigkeit einer Person vorgesehen,
a) wobei eine Menge von zumindest zwei möglichen unterschiedlich wahrnehmbare Arten von auf den Probanden applizierbaren Stimuli vorgegeben wird, und
b) dem Probanden gedankliche Tätigkeiten aufgetragen werden, die bei Vorliegen eines Stimulus abhängig von der Art dieses Stimulus ausgeführt werden sollen,
c) wobei eine Mehrzahl von Testschritten durchgeführt wird, wobei für jeden der Testschritte jeweils
   - eine Art von Stimulus aus der Menge der möglichen Arten von Stimuli, insbesondere nach Zufallskriterien, ausgewählt wird,
   - ein Stimulus der jeweils ausgewählten Art von Stimuli der Person appliziert wird,
   - innerhalb eines Zeitbereichs vor, während oder nach der Applikation des jeweiligen Stimulus EEG-Daten der Person ermittelt und aufgenommen werden, wobei der Zeitbereich vorzugsweise eine Dauer von 1 bis 10 Sekunden aufweist, und
   - die jeweils ermittelten EEG-Daten oder daraus abgeleiteten Daten der jeweiligen Art von Stimulus zugeordnet werden,
d) wobei mittels Klassifikationsanzanalyse ein Maß dafür ermittelt wird, ob die einem bestimmten Stimulus zugeordneten EEG-Daten von den einem Stimulus unterschiedlicher Art zugeordneten EEG-Daten unterscheidbar sind, und
e) wobei das Maß für die Unterscheidbarkeit der EEG-Daten unterschiedlicher Stimuli als Maß für die Wahrnehmungsfähigkeit herangezogen wird.

Die Erfindung hat den wesentlichen Vorteil, dass die Wahrnehmungsfähigkeit einer Person unabhängig davon quantifizierbar ist, ob diese dazu in der Lage ist, bestimmte motorische Aktionen tatsächlich auszuführen. Darüber hinaus ist es von Vorteil, dass der vorliegende Test einfach an unterschiedliche Testbedingungen angepasst werden kann, indem der Person unterschiedliche gedankliche Tätigkeiten aufgetragen werden, die jeweils zu unterschiedlichen Ergebnissen in den EEG-Daten führen. Je nach Person können auch individuell unterschiedliche gedankliche Tätigkeiten für die Quantifizierung herangezogen werden, um einen möglichst aussagekräftigen Wert zu erhalten. Auch kann der Test an unterschiedliche zusätzliche sensorische Störungen der Person angepasst werden.

Eine einfache Methode zur Bestimmung des Maßes sieht vor, dass das Maß ermittelt wird, indem untersucht wird, mit welcher Wahrscheinlichkeit die Anwendung der Klassifikationsanalyse auf die einzelnen den Arten von Stimuli zugeordneten EEG-Daten jeweils auf den korrekten Stimulus hindeutet.

Eine besonders einfache Ausführungsform der Erfindung, die lediglich die Funktionsfähigkeit des Gehörs voraussetzt, sieht vor, dass die Menge der Arten von Stimuli durch unterschiedliche Töne, insbesondere mit unterschiedlicher Dauer, Frequenz und Lautstärke, in für einen Menschen hörbaren Frequenzen vorgegeben wird und der jeweilige Ton der Person vorgespielt wird.

Die Erfindung, die ein geringfügiges taktiles Empfinden voraussetzt, sieht vor, dass die Menge der Arten von Stimuli Vibrationsbeaufschlagungen an unterschiedlichen Körperteilen und/oder mit unterschiedlicher Intensität und/oder Dauer umfasst, die der Person mittels Vibrationseinheiten appliziert werden.

Eine weitere Ausführungsform der Erfindung, die ein visuelles Empfinden voraussetzt, sieht vor, dass die Menge der Arten von Stimuli visuelle Reize für ein Auge oder beide Augen und/oder mit unterschiedlicher Intensität und/oder Dauer umfasst, die der Person mittels eines Bildschirms oder mittels Leuchtmitteln appliziert werden.

Eine weitere Ausführungsform der Erfindung, die ein elektrisches Reizempfinden voraussetzt, sieht vor, dass die Menge der Arten von Stimuli elektrische Reize an unterschiedlichen Körperteilen und/oder mit unterschiedlicher Intensität und/oder Dauer umfasst, die der Person mittels elektrischer Stimulatoren appliziert werden.

Besonders unterscheidungskräftige und von Probanden leicht durchführbare gedankliche Tätigkeiten, die im Zusammenhang mit der vorliegenden Erfindung besonders aussagekräftige Resultate erzielen, sind beispielsweise:
- Zählen oder Rechnen,
- Denken an Bewegungen von Körperteilen, insbesondere Extremitäten der rechten oder linken Körperhälfte, vorzugsweise der Arme oder Hände.

Eine besonders vorteilhafte Vorverarbeitung der EEG-Daten umfassend eine Vielzahl von EEG-Signalen und EEG-Kanälen sieht vor, dass eine Bewertung der aufgenommenen EEG-Daten vorgenommen wird, indem die einzelnen zum selben Zeitpunkt aufgenommenen EEG-Daten der einzelnen EEG-Kanäle jeweils zu einem Signalvektor zusammengefasst werden,
- dass eine Anzahl von, insbesondere vier, Gewichtvektoren vorgegeben wird, die dieselbe Anzahl von Elementen aufweisen wie die Signalvektoren,
- dass für jeden Zeitpunkt jeweils das Skalarprodukt des ermittelten Signalvektors mit jedem der Gewichtsvektoren erstellt wird und die jeweils erstellten Skalarprodukte dem jeweiligen Gewichtsvektor zugeordnet werden,
- dass unter den jeweils demselben Gewichtsvektor zugeordneten Skalarprodukten jeweils die Varianz über einen vorgegebenen Zeitbereich ermittelt wird und dem Gewichtsvektor zugeordnet wird,
- dass die einzelnen Varianzen und gegebenenfalls noch ein weiterer, vorgegebener Summand mit Gewichtswerten eines weiteren Gewichtsvektors gewichtet und summiert werden, und
- dass die so erhaltene Summe oder eine Folge von so unmittelbar hintereinander aufgenommener Summen der Klassifikationsanzanalyse als Testwert zugrundegelegt wird.

Eine besonders vorteilhafte, individuelle Anpassung an die jeweilige Person sieht vor, dass die Gewichtsvektoren und die Gewichtswerte, sowie gegebenenfalls der weitere Summand, an die jeweilige Person angepasst werden, sodass das in der Klassifikationsanzanalyse ermittelte Maß maximiert wird,
insbesondere indem ausgehend von vorgegebenen Startwerten die Gewichtsvektoren, die Gewichtswerte, sowie gegebenenfalls der weitere Summand so lange iterativ adaptiert werden, bis die Klassifikationsanzanalyse basierend auf den bereits ermittelten Testdaten ein maximales Maß für die Unterscheidbarkeit liefert.

Um Störungen der ermittelten EEG-Signale zu vermeiden und eine besonders hohe Unterscheidbarkeit zwischen den einzelnen EEG-Signalen zu erzielen, kann vorgesehen sein, dass die EEG-Daten vor der Beurteilung mittels Klassifikationsanzanalyse kanalweise einer Bandpassfilterung unterzogen werden, wobei das gefilterte Signal, insbesondere ausschließlich, Frequenzen zwischen 8 Hz und 30 Hz enthält.

Um eine vorteilhafte Interaktion mit dem zu untersuchenden Probanden zu erreichen sowie um den zu untersuchenden Probanden ein unmittelbares Feedback zu geben, kann vorgesehen sein, dass die EEG-Daten oder die der Klassifikationsanzanalyse zugrunde gelegten Daten, insbesondere die Resultate einer Mittelung, die aus den EEG-Daten abgeleiteten evozierten Potentiale oder die EEG-Daten nach der Durchführung einer ereignisbezogenen Desynchronisation oder die EEG-Daten, vorzugsweise der Person und/oder einem das Verfahren leitenden Operator, angezeigt werden.

Eine besonders rasche, einfache und effiziente Umsetzung kann vorgenommen werden, wenn das Maß dafür, ob die einem bestimmten Stimulus zugeordneten EEG-Daten von den einem Stimulus unterschiedlicher Art zugeordneten EEG-Daten unterscheidbar sind, mittels einer der folgenden Arten von Klassifikationsanzanalyse duchgeführt werden:
- Diskriminanzanalyse, insbesondere linearer Diskriminanzanalyse,
- Support vector machines,
- neuronale Netzwerke.

Um tages- und wochenbezogene Schwankungen einzelner Probanden ausscheiden zu können, kann vorgesehen sein, dass das Verfahren an mehreren, insbesondere aufeinander folgenden, Tagen, gegebenenfalls mehrfach, insbesondere mit denselben Stimuli, durchgeführt wird, wobei das Maß für die Wahrnehmungsfähigkeit der Person für jeden Tag separat ermittelt wird und das Maß, das die größte Wahrnehmungsfähigkeit indiziert, als Maß für die Wahrnehmungsfähigkeit der Person herangezogen wird.

Um nach der Durchführung der Quantifizierung der Wahrnehmungsfähigkeit mit einfachen Mitteln eine Kommunikation vornehmen zu können und die im Rahmen der Quantifizierung erzielten Daten weiter vorteilhaft nutzen zu können, kann vorgesehen sein,
- dass nach erfolgter Bestimmung des Maßes für die Wahrnehmungsfähigkeit der Person aufgetragen wird, zur Kommunikation, insbesondere zur Bejahung und Verneinung von Fragen, die zuvor verwendeten gedanklichen Tätigkeiten vorzunehmen,
- dass die Person in Beantwortung der gestellten Frage gedankliche Tätigkeiten vornimmt,
- dass innerhalb von vorgegebenen Zeitbereichen während oder nach der Frage EEG-Daten der Person ermittelt und aufgenommen werden,
- dass die jeweils aufgenommenen EEG-Daten mittels der zuvor durchgeführten Klassifikationsanzanalyse klassifiziert werden, und
- dass die jeweils ermittelten Ergebnisse der Klassifikation als Kommunikationsinhalte herangezogen und gegebenenfalls zur Verfügung gehalten werden.

Verfahren zur Durchführung der Erfindung können vorteilhaft mittels Computern durchgeführt werden. Die Erfindung betrifft auch einen Datenträger, auf dem ein Verfahren zur Durchführung eines erfindungsgemäßen Verfahrens nach einem der vorangehenden Ansprüche abgespeichert ist.

Eine beispielhafte Ausführungsform der Erfindung sowie einige vorteilhafte Weiterbildungen derselben werden im Folgenden näher dargestellt.

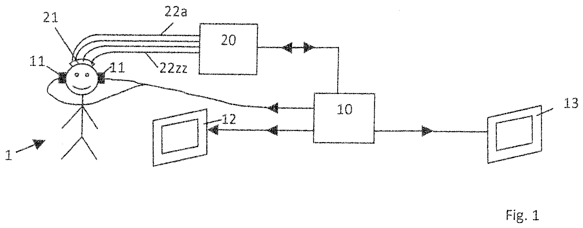

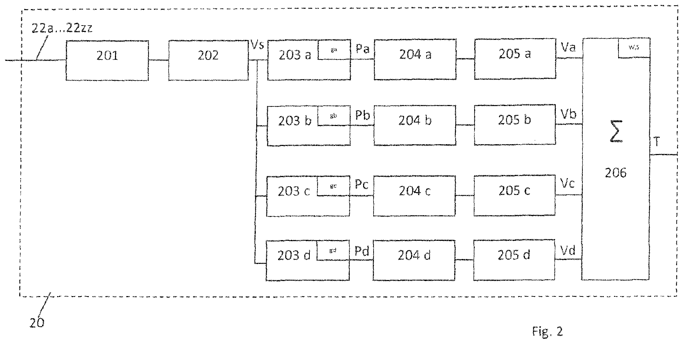

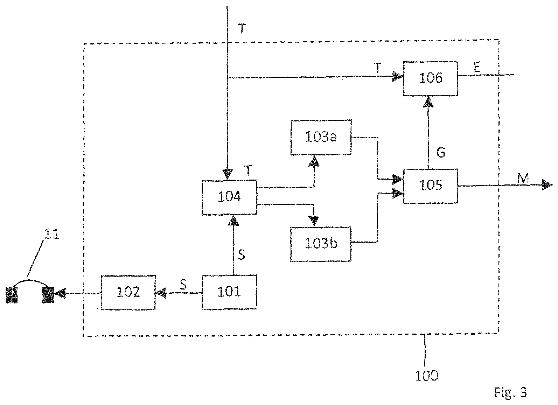

**Fig. 1** zeigt schematisch ein Beispiel einer Anordnung zur Durchführung eines erfindungsgemäßen Verfahrens. **Fig. 2** zeigt die Vorgehensweise bei der Weiterverarbeitung von EEG-Daten bis zur Ermittlung von Testwerten. **Fig. 3** zeigt die in **Fig. 1** dargestellte Steuereinheit im Detail.

**Fig. 1** zeigt eine Person, im folgenden als Probanden 1 bezeichnet, dessen Wahrnehmungsfähigkeit quantifiziert werden soll. Hierfür wurde ihm eine EEG-Haube 21 aufgesetzt, die mittels jeweils einer EEG-Kabelverbindungen 22a, ..., 22zz mit einer Testeinheit 20 verbunden sind. Weiters wurden dem Probanden 1 Kopfhörer 11 aufgesetzt, mittels denen akustische Stimuli S, beispielsweise in Form von Tönen oder Tonfolgen, auf den Probanden 1 applizierbar sind. Die Kopfhörer 11 sowie die Testeinheit 20 sind mit einer Steuereinheit 10 verbunden, die die Abgabe der Stimuli S steuert und die Testwerte der Testeinheit 20 übermittelt erhält. Mit der Steuereinheit 10 kann die Testeinheit 20 konfiguriert und an den jeweiligen Probanden 1 angepasst werden.

Zu Beginn des Verfahrens wird eine Menge von unterschiedlichen Stimuli S festgelegt. In einer bevorzugten Ausführungsform der Erfindung werden als Stimuli S Töne mit unterschiedlicher Tonhöhe festgelegt, die mittels eines Lautsprechers 11 oder Kopfhörers 11 dem Probanden 1 vorspielbar sind. Um die Quantifiziertung für den Probanden 1 möglichst einfach zu gestalten, werden im exemplarisch dargestellten Ausführungsbeispiel lediglich zwei unterschiedliche Tonhöhen als mögliche Stimuli S vorgegeben.

Selbstverständlich können im Rahmen der Erfindung jedoch auch andere Mengen von Stimuli S herangezogen werden.

Einerseits ist es möglich, dass der Proband 1 bestimmte Merkmale von Geräuschen wie Dauer, Frequenz und Lautstärke nur eingeschränkt wahrnehmen kann. Die Menge der Arten von Stimuli S kann somit auch festgelegt werden, indem unterschiedliche Töne, insbesondere mit unterschiedlicher Dauer, Frequenz und Lautstärke, in für einen Menschen hörbaren Frequenzen vorgegeben werden und der jeweilige Ton dem Probanden vorgespielt wird.

Andrerseits kann es zur genaueren Quantifizierung durchaus von Vorteil sein, mehr als zwei unterschiedliche Stimuli S zu verwenden. Auch kann bei einer bekannten entsprechenden Schädigung des Probanden 1 auch der Fall eintreten, dass dieser den Stimulus S aus anderen Gründen nicht wahrnehmen kann, zum Beispiel besteht die Möglichkeit, dass der Proband 1 zwar bei vollem Bewusstsein ist, jedoch eine Störung der Gehörgänge oder des Hörnervs aufweist und aus diesem Grund nicht in der Lage ist, einen Hörreiz als Stimulus S adäquat zu interpretieren.

Es ist daher auch möglich, andere visuell, elektrisch und taktil oder sonst wahrnehmbare Reize als Stimuli S zu verwenden. Die Menge der Arten von Stimuli S können beispielsweise in Form von Vibrationsbeaufschlagungen an unterschiedlichen Körperteilen und/oder mit unterschiedlicher Intensität und/oder Dauer dem Probanden mittels Vibrationseinheiten appliziert werden.

In einem weiteren zu Beginn des Verfahrens vorzunehmenden Schritt wird dem Probanden 1 mitgeteilt, welche Reaktionen er als Antwort für die jeweiligen Stimuli S vorzunehmen hat. Diese Mitteilung kann auf unterschiedliche Art und Weise erfolgen, beispielsweise durch Erklärung in Form einer Sprachmitteilung oder durch Darstellung der gewünschten Vorgehensweise auf einem Bildschirm 12.

Ein möglicher Auftrag an den Probanden 1 ist beispielsweise die Anweisung, bei einem hohen Ton an die rechte Hand und bei einem tiefen Ton an die linke Hand zu denken. Alternativ kann ein möglicher Auftrag darin bestehen, bei Vorliegen eines taktilen Reizes durch eine Vibrationseinheit in einem vorgegebenen Körperbereich gedanklich zu zählen. Angepasst an unterschiedliche Fähigkeiten des Probanden 1 sowie das Wissen um sensorische Vorbeeinträchtigungen kann ein an den Probanden 1 angepasster Auftrag erstellt werden.

Je nach Fortschreiten des Quantifizierungsverfahrens können auch differenziertere gedankliche Tätigkeiten verlangt werden, beispielsweise das Denken an einen von mehreren vorab vorgegebenen Körperbereichen als Reaktion auf jeweils einen Stimulus S aus einer vorgegebenen Menge von Stimuli S.

Vor der Durchführung einzelner Testschritte des Verfahrens wird eine Anzahl von EEG-Elektroden auf den Kopf des Probanden 1 aufgesetzt. Auf den Kopf eines Probanden 1 wird eine Probenanordnung mit Elektroden, im vorliegenden Beispiel mit 27 Elektroden, aufgebracht. Die einzelnen von den Elektroden ermittelten Ableitungen werden an eine Verstärkereinheit 201 geführt, verstärkt und digitalisiert.

Vor der Durchführung des Tests werden die einzelnen Stimuli S auf den Probanden 1 appliziert, um diesen mit sämtlichen Stimuli S vertraut zu machen. Im vorliegenden Beispiel werden dem Probanden 1 sowohl der hohe als auch der tiefe Ton vorgespielt und anschließend wird ihm mitgeteilt, welche Reaktion von ihm erwartet wird, nämlich,
- dass er bei Erkennen eines hohen Tons an die Extremitäten seiner rechten Hand oder eine Bewegung der Extremitäten der rechten Hand denken soll und
- dass er bei Erkennen eines tiefen Tons an die Extremitäten seiner linken Hand oder eine Bewegung der Extremitäten der linken Hand denken soll.

Im vorliegend beschriebenen Verfahren werden an drei aufeinander folgenden Tagen jeweils 50 Testschritte hintereinander durchgeführt. Hierbei wird in jedem einzelnen Testschritt nach Zufallskriterien jeweils eine Art von Stimulus S aus der Menge der Stimuli S ausgewählt. Eine Zufallseinheit 101 (Fig. 3) wählt eine Art von Stimulus S aus und übermittelt ein diesbezügliches Auswahlsignal an eine Stimulus-Einheit 102, die den Stimulus S in Form eines elektrischen Analogsignals an die Kopfhörer 11 überträgt.

Es besteht hierbei die Möglichkeit, das Maß für die Wahrnehmungsfähigkeit des Probenden 1 an mehreren Tagen separat für den jeweiligen Tag zu ermitteln und das Maß, das die größte Wahrnehmungsfähigkeit indiziert, als Maß für die Wahrnehmungsfähigkeit des Probanden insgesamt heranzuziehen.

Im vorliegenden Ausführungsbeispiel wurde im ersten Testschritt dem Probanden 1 für eine Sekunde ein hoher Ton vorgespielt. Der Proband 1 erkennt den hohen Ton als solchen und denkt während des Abspielens des hohen Tons oder danach entsprechend an seine rechte Hand. Innerhalb eines Zeitfensters von 10 Sekunden werden sämtliche von den EEG-Kanäle für eine weitere Untersuchung herangezogen. Der Beginn dieses Zeitfensters kann vor, während oder nach dem Stimulus liegen. Im vorliegenden Beispiel beginnt das Zeitfenster 100 ms vor dem Beginn des Stimulus.

Die einzelnen EEG-Signale werden mit einer Abtastrate von 256 Abtastungen pro Sekunde mit einem Analog-Digital-Konverter 201 (Fig. 2) abgetastet und in Digitalsignale konvertiert, sodass sich insgesamt zur Charakterisierung der Gedanken des Probanden 256x10x27=69.120 über 10 Sekunden einzelne Abtastwerte ergeben.

Die aus der EEG-Messung gewonnenen Abtastwerte werden kanalweise einer Bandpassfilterung 202 unterzogen. Es wird dabei vor oder nach der Abtastung eine Filterung vorgenommen, dass Frequenzanteile des Signals, die kleiner als 8 Hz und größer als 30 Hz sind, stark gedämpft werden.

Grundsätzlich können einzelne aus der Gesamtheit der Signale abgeleitete Werte, beispielsweise eines Signalvektors s umfassend alle einzelnen kanalweise bestimmten Signalwerte des EEG-Signals, für eine Diskriminanzanalyse herangezogen werden. Eine solcherart durchgeführte Diskriminanzanalyse kann prinzipiell zur Quantifizierung der Wahrnehmungsfähigkeit weiter genutzt werden.

Die vorliegende bevorzugte Ausführungsform der Erfindung sieht jedoch eine Vereinfachung vor, die eine Ausführung des Verfahrens mit wesentlich geringerem Ressourcenverbrauch ermöglicht. Hierfür werden sämtliche zum selben Zeitpunkt aufgenommenen Signalwerte der einzelnen EEG-Kanäle zu einem gemeinsamen Signalvektor s zusammengefasst. Im vorliegenden Beispiel umfassen die Signalvektoren s jeweils 27 einzelnen Signalwerte, nämlich je einen pro EEG-Kanal.

Weiters werden für die jeweilige Person vier individuelle Gewichtsvektoren gₐ, ..., g_{d} ermittelt, die dieselbe Größe aufweisen wie die Signalvektoren s. Im vorliegenden Beispiel weisen die Gewichtsvektoren jeweils 27 Elemente oder Einträge auf. Für jeden einzelnen Aufnahme- bzw. Abtastzeitpunkt während des Zeitfensters wird von jeweils einer Gewichtungseinheit 203a, 203b, 203c, 203d jeweils ein Skalaprodukt pₐ, p_{b}, p_{c}, p_{d} des ermittelten Signalvektors s mit jedem der Gewichtsvektoren gₐ, ..., g_{d} erstellt. In dieser vorteilhaften Ausführungsform der Erfindung werden in nachgelagerten Pufferspeichern 204a, 204b, 204c, 204d jeweils die zuletzt erstellten, beispielsweise 5 bis 100, Skalaprodukte pₐ, p_{b}, p_{c}, p_{d}. separat für den jeweiligen Gewichtsvektor gₐ, ..., g_{d} gespeichert. Anschließend wird für diese in den Pufferspeichern 204a, 204b, 204c, 204d liegenden Skalaprodukte pₐ, p_{b}, p_{c}, p_{d} jeweils die Varianz vₐ, v_{b}, v_{c}, v_{d} ermittelt. Diese alle zu einem Zeitpunkt ermittelten Varianzen vₐ, v_{b}, v_{c,} v_{d} werden jeweils an eine nachgeschaltete Gewichtungseinheit 206 übermittelt. Diese Gewichtungseinheit 206 bildet eine gewichtete Summe der einzelnen Varianzen vₐ, v_{b}, v_{c,} v_{d} wobei jede der Varianzen jeweils mit einem Gewichtswert w_{a,} w_{b}, w_{c}, w_{d} aus einem weiteren Gewichtsvektor **w** herangezogen werden. Die Gewichtungseinheit 206 addiert gegebenenfalls noch einen weiteren Summanden s hinzu, sodass am Ausgang der Gewichtungseinheit 206 ein skalarer Wert T anliegt.

Die Folge der innerhalb des Zeitfensters ermittelten Werte wird in weiterer Folge als Testwert bezeichnet und von der Testeinheit 20 an die Steuereinheit 10 übertragen und von dieser der jeweiligen Art von Stimulus S zugeordnet. Die Gewichtsvektoren gₐ, ..., g_{d}, der weitere Gewichtsvektor W und der weitere Summand S werden im Folgenden als Individualdaten bezeichnet und für jeden Probanden 1 separat ermittelt.

Die konkrete Ermittlung der Individualdaten gₐ, ..., g_{d}, **w**, s erfolgt im vorliegenden Ausführungsbeispiel in einem Optimierungsverfahren und wird unten näher beschrieben und erfolgt vorteilhafterweise nach der Durchführung weniger Testschritte und kann gegebenenfalls zur Anpassung an Trainingserfolge des Probanden 1 wiederholt werden.

Mit diesem vorteilhaften Vorgehen kann gewährleistet werden, dass die Diskriminanzanalyse mit numerisch geringem Aufwand vorgenommen werden kann. Insbesondere brauchen nicht 20250 Skalarwerte sondern bloß ein Testwert T mit einer geringen Anzahl von Skalarwerten ausgewertet werden, wodurch eine drastisch vereinfachte Diskriminanzanalyse vorgenommen werden kann. Insbesondere bei einer nachträglichen Weiterverwendung der Erfindung für die Kommunikation nach Abschluss der Quantifizierung der Wahrnehmungsfähigkeit kann auf diese Weise eine beträchtliche Ressourcenersparnis erreicht werden.

Der im jeweiligen Testschritt gewonnen Testwerte T werden der jeweiligen Art des Stimulus S zugeordnet. Im vorliegenden Beispiel wurde mit den Kopfhörern 11 ein hoher Ton als Stimulus S vorgespielt. Die Art des Stimulus wird an eine Speichersteuerungseinheit 104 übertragen, die den Testwert T an einen ersten Speicher 103a zur Abspeicherung weiterleitet. Wird in einem zweiten oder weiteren Testschritt ein tiefer Ton als Stimulus S vorgegeben, so leitet die Speichersteuerungseinheit 104 den Testwert T an einen zweiten Speicher 103b weiter. Am Ende der einzelnen Testschritte liegen in den beiden Speichern 103a, 103b jeweils eine Vielzahl unterschiedlicher Testwerte T vor.

Mittels Diskriminanzanalyse 105, der die einzelnen in den Speichern 103a, 103b abgelegten Testwerte T zugeführt sind, kann ein Unterscheidungskriterium G ermittelt werden, das eine Unterscheidung zwischen den Testwerten T, die von den einzelnen Arten von Stimuli S, beispielsweise einem hohen Ton und Testwerten, die von einem tiefen Ton herrühren, ermöglicht. Darüber hinaus liefert die Diskriminanzanalyse 105 auch ein Maß M für die Unterscheidbarkeit der zu separierenden Testwerte T, das aus den nachstehenden Gründen als Maß M für die Wahrnehmungsfähigkeit des Probanden 1 angesehen wird.

Führt beispielsweise ein nach einem hohen Ton vom Probanden 1 durch Denken an die rechte Hand erzeugtes EEG-Signal immer zu einem Testwert T, der dem Unterscheidungskriterium G entspricht und ein nach einem tiefen Ton vom Probanden 1 durch Denken an die linke Hand erzeugtes Signal immer zu einem Testwert T, der dem Unterscheidungskriterium nicht entspricht, so liefert die Diskriminanzanalyse 105 ein maximales Maß M für die Unterscheidbarkeit. Es kann in diesem Fall davon ausgegangen werden, dass der betreffende Proband 1 den Auftrag verstanden hat und die einzelnen applizierten Stimuli S wahrnehmen konnte und auf diese zielgerichtet reagiert hat.

Sofern der jeweilige Proband 1 jedoch überhaupt keine Wahrnehmungsfähigkeit hat, können auch keine unterschiedlichen gedanklichen Tätigkeiten festgestellt werden. Auch wenn die Diskriminanzanalyse 105 einen Unterscheidungskriterium G liefert, so kann aus dem Erfüllen oder Nichterfüllen des Unterscheidungskriteriums G durch den jeweiligen Testwert T kein Rückschluss auf den jeweiligen Stimulus S gezogen werden. Das Maß M für die Unterscheidbarkeit der einzelnen Testwerte ist folglich gering.

Zur Ermittlung der Individualwerte gₐ, ..., g_{d}, **w**, s, die für die Ermittlung der Testwerte T verwendet werden, kann wie folgt vorgegangen werden: Ausgehend von zufällig vorgegebenen Startwerten für die Individualwerte gₐ, ..., g_{d}, **w**, s oder ausgehend von den Startwerten von bereits getesteten Probanden 1 mit hoher Wahrnehmungsfähigkeit können die Individualdaten, nämlich die Gewichtsvektoren gₐ, ..., g_{d}, die weiteren Gewichtsvektoren **w** und der weitere Summand s, so lange adaptiert werden, bis die Diskriminanzanalyse 105, basierend auf einem ersten Satz von EEG-Daten einen ein maximales Maß für die Unterscheidbarkeit liefert. Der erste Satz von EEG-Daten muss nicht notwendigerweise sämtliche vom Probanden 1 erfassten EEG-Daten enthalten. Es ist durchaus möglich, dass nur ein Teil der EEG-Daten zur Ermittlung der Individualdaten gₐ, ..., g_{d}, **w**, s herangezogen wird. Um Anpassungseffekte der Probanden 1 an die jeweilige Aufgabe besser berücksichtigen zu können, können die Individualdaten gₐ, ..., g_{d}, **w**, s auch nach bestimmten Zeitintervalle neu bestimmt werden. Dies ist insbesondere dann von Vorteil, wenn das vorgestellte Verfahren nach der Quantifizierung der Wahrnehmungsfähigkeit weiter zur Kommunikation benutzt wird.

Dieser Vorgang der Anpassung der Individualdaten gₐ, ..., g_{d}, **w**, s kann iterativ so lange wiederholt werden bis eine optimale Unterscheidbarkeit der Testwerte vorliegt.

Zur Optimierung der Individualwerte gₐ, ..., g_{d}, **w**, s kann prinzipiell jedes Optimierungsverfahren verwendet werden. Gute Ergebnisse haben die Erfindung mit einem Verfahren erzielt, das aus der Literatur bekannt ist und dessen Inhalt in diese Anmeldung aufgenommen wird:
Guger, C.; Ramoser, H.; Pfurtscheller, G., "Real-time EEG analysis with subject-specific spatial patterns for a brain-computer interface (BCI)," Rehabilitation Engineering, IEEE Transactions on, vol.8, no.4, pp.447,456, Dec 2000 doi: 10.1109/86.895947

Das Optimierungsverfahren kann von einer nicht dargestellten Optimierungseinheit der Steuereinheit 10 gesteuert werden, die die einzelnen Individualwerte gₐ, ..., g_{d}, **w**, s abändert und iterativ jeweils eine neuerliche Erstellung der Testdaten T durch die Testeinheit 20 startet.

Auch zur Diskriminanzanalyse 105 können unterschiedliche numerische Verfahren verwendet werden, wobei sich im vorliegenden Fall das in *"*C. M. Bishop, Neural Networks for Pattern Recognition, 1995 :Clarend*on"* beschriebene Verfahren zur linearen Diskriminanzanalyse besonders eignet.

Um auch während des Quantifizierungsverfahrens eine visuelle Kontrolle durch den Probanden 1 oder durch Dritte zu ermöglichen, kann nach dem jeweiligen Testschritt der im Testschritt ermittelte Testwert T von einer Vergleichseinheit 106 dem aus der Diskriminanzanalyse 105 ermittelten Unterscheidungskriterium G unterzogen werden. Das Ergebnis E der Anwendung des Kriteriums G auf den Testwert T kann anschließend dem Probanden 1 oder einem Dritten angezeigt oder sonst zur Kenntnis gebracht werden. Das Ergebnis kann beispielsweise auf Bildschirmen 12, 13 graphisch dargestellt werden. Nach einer Anzahl von Testschritten kann auch die Diskriminanzanalyse erneut durchgeführt und das Unterscheidungskriterium G neu festgelegt werden.

Die Ermittlung des Unterscheidungskriteriums G ist grundsätzlich für die Quantifizierung des Wahrnehmungsfähigkeit nicht erforderlich, kann aber zur Bestimmung der Reaktion des Probanden 1 im Einzelfall verwendet werden.

Diesen Umstand macht sich eine besondere Weiterbildung der Erfindung zunutze, die nach der Quantifizierung des Wahrnehmungsfähigkeit des Probanden 1 zusätzlich eine ständige Kommunikation mit dem Probanden 1 anstrebt.

Nach der Quantifizierung der Wahrnehmungsfähigkeit des Probanden 1 kann diesem zu Zwecken der Kommunikation weitere Fragen gestellt werden und des Probanden 1 gedankliche Tätigkeiten aufgetragen werden, die bei Bejahung oder Verneinung dieser Frage ausgeführt werden sollen. Diese Fragen können beispielsweise dem Probanden 1 auf ihrem Bildschirm 12 dargestellt werden oder über die Kopfhörer 11 übertragen werden.

Hierbei verwendet der Proband 1 als Antwort dieselben gedanklichen Tätigkeiten, die zuvor bei der Quantifizierung der Wahrnehmungsfähigkeit verwendet wurden, da hinsichtlich dieser gedanklichen Tätigkeiten bereits eine ausreichende Unterscheidbarkeit sowie ein Unterscheidungskriterium G zur Unterscheidung der Antworten bzw. der Testwerte T festgestellt wurde. Bei den an den Probanden 1 gerichteten Fragen handelt es sich normalerweise um Fragen, die mit vorgegebenen Antwortmöglichkeiten ausgestattet sind, beispielsweise die Antworten "JA" und "NEIN". Auch ist es möglich, mit dem Probanden 1 ein komplexeres Antwortschema mit einer größeren Anzahl von unterscheidbaren gedanklichen Tätigkeiten als Antworten zu vereinbaren, wenn diese zuverlässig und unterscheidbar festgestellt werden können.

Der Proband 1 nimmt im Anschluss an einzelne Fragen gedankliche Tätigkeiten vor, die vom EEG erfasst und mittels der zuvor ermittelten Diskriminanzanalyse 105 klassifiziert werden, d.h. es wird das der Diskriminanzanalyse 105 entstammende Unterscheidungskriterium G auf den Testwert angewendet und das Ergebnis E ermittelt. Abhängig vom Ergebnis E, wird eine unterschiedliche Antwort des Probanden 1 auf die gestellte Frage angenommen. Die Antworten bzw. die ermittelten Ergebnisse E der Klassifikation werden für den Fragensteller zur Verfügung gehalten und diesem sowie gegebenenfalls auch dem Probanden auf den Bildschirmen 12, 13 angezeigt.

Im vorstehenden Ausführungsbeispiel der Erfindung wurde beispielhaft eine lineare Diskriminanzanalyse zur Ermittlung des Maßes M für die Unterscheidbarkeit herangezogen. Dies ist jedoch nicht zwingend erforderlich. Vielmehr können unterschiedliche Arten der Klassifikationsanalyse, mit denen eine Trennung von unterschiedlichen Testwerten möglich ist, herangezogen werden. So können beispielsweise auch Support vector machines *"*Hidden Markov model and support vector machine based decoding of finger movements using electrocorticography; Wissel T, Pfeiffer T, Frysch R, Knight RT, Chang EF, Hinrichs H, Rieger JW, Rose G. J Neural Eng. 2013 Oct;10(5):056020. doi: 10.1088/1741-2560/10/5/056020*. Epub 2013 Sep 18. PMID: 24045504 [PubMed* - *in process]"* oder neuronale Netzwerke *"*C. M. Bishop, Neural Networks for Pattern Recognition, 1995 :Clarend*on"* zur Klassifikationsanalyse herangezogen werden, um ein Maß M für die Unterscheidbarkeit zu bestimmen.

Das Maß M kann auch ermittelt werden, indem untersucht wird, mit welcher Wahrscheinlichkeit die Anwendung der Klassifikationsanalyse 105 auf die einzelnen den Arten von Stimuli zugeordneten EEG-Daten jeweils auf den korrekten Stimulus hindeutet. Hierzu wird jeweils die Klassifikationsanalyse 105 nachträglich auf sämtliche ermittelten EEG-Daten einzeln angewendet, wobei jeweils ein Klassifikationsergebnis ermittelt wird. Anschließend wird untersucht, ob das jeweilige Klassifikationsergebnis mit dem Stimulus übereinstimmt, der bei der Ermittlung der EEG-Daten auf den Patienten appliziert wurde. Das Verhältnis der korrekten Beurteilungen zu der Gesamtanzahl der einzelnen ermittelten EEG-Daten bzw. der Anzahl der applizierten Stimuli kann als Maß M herangezogen werden.

Evozierte Potentiale werden durch Mittelung der EEG Daten eines spezielles Stimulus berechnet und als EP-Kurve dargstellt. Die Software überlagert die evozierten Potentiale von zwei Klassen und daher können Unterschiede leicht erkannt werden. Dies erlaubt einerseits zu sehen ob der erwartete physiologische Response aufgetreten ist und weiters zu erkennen ob Unterschiede bestehen. Weiters wird ein statistischer Test durchgeführt, der angibt ob die Daten unterscheidbar sind. Statistisch signifikante Unterschiede werden in der Kurvendarstellung markiert.

Ereignisbezogenen Desynchronization wird für jede Klasse berechnet indem die Daten in einem typischen Frequenzbereich gefiltert werden (z.b. alpha bereich 8-12 Hz, beta bereich 16-24 Hz,...). Danach wird die Leistung berechnet und diese Daten über alle Wiederholungen gemittelt. Anschließend wird noch eine Mittelung im Zeitbereich durchgeführt, um die Kurven zu glätten. Diese Änderung der Leistung wird auf ein Referenzintervall vor der mentalen Aktivität bezogen und gibt daher die Änderung der Bandleistung aufgrund der durchgeführten Aktivität an. Dieses Ergebnis wird noch mit einem statistischen Test ausgewertet, sodass nur signifikante Änderungen dargestellt werden.

Sowohl Ereignisbezogenen Desynchronization aus auch evozierte Potentiale können visualisiert werden und können dem Patienten als Feedback dienen, damit er Aktivitäten besser durchführt. Für den Operator ist es wichtige Information, ob der Patient die Aufgabe richtig macht und er kann korrigierend eingreifen. Weiters kann der Operator physiologische Effekte aufgrund seiner Erfahrung beurteilen.

## Patentansprüche

1. Verfahren zur Quantifizierung der Wahrnehmungsfähigkeit einer Person (1), der keinerlei sonstige Möglichkeiten zur Kommunikation, wie insbesondere Sprache oder Gebärden, zur Verfügung stehen, sowie zur späteren Kommunikation mit dieser Person (1),
a) wobei eine Menge von zumindest zwei möglichen unterschiedlich wahrnehmbare Arten von auf den Probanden (1) applizierbaren Stimuli (S) vorgegeben wird, wobei der Person (1) Vibrationsbeaufschlagungen an unterschiedlichen Körperteilen und/oder mit unterschiedlicher Intensität und/oder Dauer mittels Vibrationseinheiten appliziert werden, und
b) dem Probanden (1) gedankliche Tätigkeiten aufgetragen werden, die bei Vorliegen eines Stimulus (S) abhängig von der Art dieses Stimulus (S) ausgeführt werden sollen,
c) wobei eine Mehrzahl von Testschritten durchgeführt wird, wobei für jeden der Testschritte jeweils
- eine Art von Stimulus (S) aus der Menge der möglichen Arten von Stimuli (S), insbesondere nach Zufallskriterien, ausgewählt wird,
- ein Stimulus (S) der jeweils ausgewählten Art von Stimuli der Person (1) appliziert wird,
- innerhalb eines Zeitbereichs vor, während oder nach der Applikation des jeweiligen Stimulus (S) EEG-Daten der Person ermittelt und aufgenommen werden, wobei der Zeitbereich vorzugsweise eine Dauer von 1 bis 10 Sekunden aufweist, und
- die jeweils ermittelten EEG-Daten gemittelt und die derart ermittelten evozierten Potentiale dem jeweiligen Stimulus (S) zugeordnet werden,
d) wobei mittels Klassifikationsanalyse (105) ein Maß (M) dafür ermittelt wird, ob die einem bestimmten Stimulus (S) zugeordneten EEG-Daten von den einem Stimulus (S) unterschiedlicher Art zugeordneten EEG-Daten unterscheidbar sind, und
e) wobei das Maß (M) für die Unterscheidbarkeit der EEG-Daten unterschiedlicher Stimuli (S) als Maß für die Wahrnehmungsfähigkeit herangezogen wird, die angibt, inwieweit der zur Kommunikation mit seiner Umwelt noch in der Lage ist,
- wobei nach erfolgter Bestimmung des Maßes (M) für die Wahrnehmungsfähigkeit der Person (1) aufgetragen wird, zur Kommunikation, insbesondere zur Bejahung und Verneinung von Fragen, die zuvor verwendeten gedanklichen Tätigkeiten vorzunehmen,
- wobei die Person (1) in Beantwortung der gestellten Frage gedankliche Tätigkeiten vornimmt,
- wobei innerhalb von vorgegebenen Zeitbereichen während oder nach der Frage EEG-Daten der Person (1) ermittelt und aufgenommen werden,
- wobei die jeweils aufgenommenen EEG-Daten mittels der zuvor durchgeführten Klassifikationsanalyse (105) klassifiziert werden, und
- wobei die jeweils ermittelten Ergebnisse (E) der Klassifikation als Kommunikationsinhalte herangezogen und gegebenenfalls zur Verfügung gehalten werden.

2. Verfahren nach Anspruch 1, **dadurch gekennzeichnet, dass** das Maß (M) ermittelt wird, indem untersucht wird, mit welcher Wahrscheinlichkeit die Anwendung der Klassifikationsanalyse (105) auf die einzelnen den Arten von Stimuli zugeordneten EEG-Daten jeweils auf den korrekten Stimulus hindeutet.

3. Verfahren nach Anspruch 1 oder 2, **dadurch gekennzeichnet,**
- **dass** die Menge der Arten von Stimuli (S) durch unterschiedliche Töne, insbesondere mit unterschiedlicher Dauer, Frequenz und Lautstärke, in für einen Menschen hörbaren Frequenzen vorgegeben wird und der jeweilige Ton der Person (1) vorgespielt wird,

4. Verfahren nach einem der vorangehenden Ansprüche, **dadurch gekennzeichnet, dass** die Menge der Arten von Stimuli (S) visuelle Reize für ein Auge oder beide Augen und/oder mit unterschiedlicher Intensität und/oder Dauer umfasst, die der Person (1) mittels eines Bildschirms oder mittels Leuchtmitteln appliziert werden.

5. Verfahren nach einem der vorangehenden Ansprüche, **dadurch gekennzeichnet, dass** die Menge der Arten von Stimuli (S) elektrische Reize an unterschiedlichen Körperteilen und/oder mit unterschiedlicher Intensität und/oder Dauer umfasst, die der Person (1) mittels elektrischer Stimulatoren appliziert werden.

6. Verfahren nach einem der vorangehenden Ansprüche, **dadurch gekennzeichnet, dass** die Person (1) abhängig vom jeweiligen Art von Stimulus (S) eine der folgenden gedanklichen Tätigkeiten aufgetragen wird, insbesondere bei Vorliegen eines taktilen Reizes durch eine Vibrationseinheit in einem vorgegebenen Körperbereich:
- Zählen oder Rechnen,
- Denken an Bewegungen von Körperteilen, insbesondere Extremitäten der rechten oder linken Körperhälfte, vorzugsweise der Arme oder Hände.

7. Verfahren nach einem der vorangehenden Ansprüche, **dadurch gekennzeichnet, dass** eine Bewertung der aufgenommenen EEG-Daten vorgenommen wird, indem die einzelnen zum selben Zeitpunkt aufgenommenen EEG-Daten der einzelnen EEG-Kanäle jeweils zu einem Signalvektor (s) zusammengefasst werden,
- dass eine Anzahl von, insbesondere vier, Gewichtvektoren (gₐ, g_{b}, g_{c}, g_{d}) vorgegeben wird, die dieselbe Anzahl von Elementen aufweisen wie die Signalvektoren (s),
- dass für jeden Zeitpunkt jeweils das Skalarprodukt (p_{a,} p_{b}, p_{c}, p_{d}) des ermittelten Signalvektors (s) mit jedem der Gewichtsvektoren (gₐ, g_{b}, g_{c}, g_{d}) erstellt wird und die jeweils erstellten Skalarprodukte (p_{a,} p_{b}, p_{c}, p_{d}) dem jeweiligen Gewichtsvektor (gₐ, g_{b}, g_{c}, g_{d}) zugeordnet werden,
- dass unter den jeweils demselben Gewichtsvektor (gₐ, g_{b}, g_{c}, g_{d}) zugeordneten Skalarprodukten jeweils die Varianz (vₐ, v_{b}, v_{c}, v_{d}) über einen vorgegebenen Zeitbereich ermittelt wird und dem Gewichtsvektor zugeordnet wird,
- dass die einzelnen Varianzen (vₐ, v_{b}, v_{b}, v_{d}) und gegebenenfalls noch ein weiterer, vorgegebener Summand mit Gewichtswerten (wₐ, w_{b}, w_{c}, w_{d}) eines weiteren Gewichtsvektors (w) gewichtet und summiert werden, und
- dass die so erhaltene Summe oder eine Folge von so unmittelbar hintereinander aufgenommener Summen der Klassifikationsanzanalyse (105) als Testwert (T) zugrundegelegt wird.

8. Verfahren nach Anspruch 7, **dadurch gekennzeichnet, dass** die Gewichtsvektoren (gₐ, g_{b}, g_{c}, g_{d}) und die Gewichtswerte (wₐ, w_{b}, w_{c}, w_{d}), sowie gegebenenfalls der weitere Summand (s), an die jeweilige Person (1) angepasst werden, sodass das in der Klassifikationsanzanalyse (105) ermittelte Maß (M) maximiert wird,
insbesondere indem ausgehend von vorgegebenen Startwerten die Gewichtsvektoren (gₐ, g_{b}, g_{c}, g_{d}), die Gewichtswerte (wₐ, w_{b}, w_{c}, w_{d}), sowie gegebenenfalls der weitere Summand (s) so lange iterativ adaptiert werden, bis die Klassifikationsanzanalyse (105) basierend auf den bereits ermittelten Testdaten ein maximales Maß (M) für die Unterscheidbarkeit liefert.

9. Verfahren nach Anspruch 7 oder 8, **dadurch gekennzeichnet, dass** die EEG-Daten vor der Beurteilung mittels Klassifikationsanzanalyse kanalweise einer Bandpassfilterung (201) unterzogen werden, wobei das gefilterte Signal (s), insbesondere ausschließlich, Frequenzen zwischen 8 Hz und 30 Hz enthält.

10. Verfahren nach einem der vorangehenden Ansprüche, **dadurch gekennzeichnet, dass** die EEG-Daten oder die der Klassifikationsanalyse (105) zugrunde gelegten Daten, insbesondere die Resultate einer Mittelung, die aus den EEG-Daten abgeleiteten evozierten Potentiale oder die EEG-Daten nach der Durchführung einer ereignisbezogenen Desynchronisation oder die EEG-Daten, vorzugsweise der Person (1) und/oder einem das Verfahren leitenden Operator, angezeigt werden oder mit einem statistischen Test ausgewertet wird und nur signifikante Änderungen angezeigt werden.

11. Verfahren nach einem der vorangehenden Ansprüche, **dadurch gekennzeichnet, dass** das Maß (M) dafür, ob die einem bestimmten Stimulus (S) zugeordneten EEG-Daten von den einem Stimulus (S) unterschiedlicher Art zugeordneten EEG-Daten unterscheidbar sind, mittels einer der folgenden Arten von Klassifikationsanzanalyse duchgeführt werden:
- Diskriminanzanalyse, insbesondere linearer Diskriminanzanalyse,
- Support vector machines,
- neuronale Netzwerke.

12. Verfahren nach einem der vorangehenden Ansprüche, **dadurch gekennzeichnet, dass** das Verfahren an mehreren, insbesondere aufeinander folgenden, Tagen, gegebenenfalls mehrfach, insbesondere mit denselben Stimuli (S), durchgeführt wird, wobei das Maß (M) für die Wahrnehmungsfähigkeit der Person (1) für jeden Tag separat ermittelt wird und das Maß (M), das die größte Wahrnehmungsfähigkeit indiziert, als Maß (M) für die Wahrnehmungsfähigkeit der Person (1) herangezogen wird.

## Claims

1. A method for quantifying the perceptive faculty of a person (1) who has no other means of communication, such as, in particular, speech or gestures, available and for subsequent communicating with said person (1),
a) wherein a quantity of at least two possible differently perceptible types of stimuli (S) which can be applied to the test persons (1) is predetermined, wherein vibrations are applied to the person (1) to different body parts and/or with different intensity and/or duration by means of vibrational units, and
b) the test person (1) is given mental tasks to be performed in the presence of a stimulus (S) according to the type of said stimulus (S),
c) wherein a plurality of test steps are performed, wherein for each of the test steps, respectively
- a type of stimulus (S) is selected in each case from the quantity of possible types of stimuli (S), in particular according to random criteria,
- a stimulus (S) of the selected type of stimuli is applied to the person (1),
- EEG data of the person are determined and recorded within a time range before, during or after the application of the stimulus (S), wherein the time range preferably has a duration of 1 to 10 seconds, and
- the EEG data determined in each case are averaged and the evoked potentials thus determined are associated with the stimulus (S),
d) wherein a measure (M) is determined by means of classification analysis (105) for whether the EEG data associated with a certain stimulus (S) can be distinguished from the EEG data associated with a stimulus (S) of a different type, and
e) wherein the measure (M) for the distinguishability of the EEG data of different stimuli (S) is used as a measure of the perceptive faculty, indicating to what extent the person is still capable of communicating with his or her environment,
- wherein after the measure (M) of the perceptive faculty of the person (1) has been determined, the previously used mental tasks are to be performed for communication, in particular for affirming and denying questions,
- wherein the person (1) carries out mental tasks in answer to the question asked,
- wherein EEG data of the person (1) are determined and recorded within predetermined time ranges during or after the question,
- wherein the EEG data recorded in each case are classified by means of the previously performed classification analysis (105) and
- the results (E) of the classification which are determined in each case are used as communication content and, optionally, kept available.

2. The method according to claim 1, **characterised in that** the measure (M) is determined by examining the probability with which the application of the classification analysis (105) to the individual EEG data associated with the types of stimuli indicates the correct stimulus in each case.

3. The method according to claim 1 or 2, **characterised in**
- **that** the quantity of the types of stimuli (S) is predetermined by different tones, in particular having different duration, frequency and volume, in frequencies audible to a human being, and the tone is played to the person (1).

4. The method according to one of the preceding claims, **characterised in that** the quantity of types of stimuli (S) comprises visual stimulations for one or both eyes and/or of different intensity and/or duration which are applied to the person (1) by means of a screen or by means of light sources.

5. The method according to one of the preceding claims, **characterised in that** the quantity of types of stimuli (S) comprises electrical stimulations to different body parts and/or of different intensity and/or duration which are applied to the person (1) by means of electrical stimulators.

6. The method according to one of the preceding claims, **characterised in that** the person (1) is assigned one of the following mental tasks according to the type of stimulus (S), in particular in the case of a tactile stimulation by a vibrational unit in a predetermined body region:
- counting or calculating,
- thinking of movements of body parts, in particular extremities of the right or left half of the body, preferably the arms or hands.

7. The method according to one of the preceding claims, **characterised in that** the recorded EEG data is evaluated by combining each individual piece of EEG data recorded at the same time of the individual EEG channels into a signal vector (s),
- **in that** a number of, in particular four, weight vectors (gₐ, g_{b}, g_{c}, g_{d}) is predetermined which have the same number of elements as the signal vectors (s),
- **in that**, for each time, the scalar product (p_{a,} p_{b}, p_{c}, p_{d}) of the determined signal vector (s) is produced with each of the weight vectors (gₐ, g_{b}, g_{c}, g_{d}) and the scalar products (p_{a,} p_{b}, p_{c}, p_{d}) produced are associated with the respective weight vectors (gₐ, g_{b}, g_{c}, g_{d}),
- **in that** the variance (vₐ, v_{b}, v_{c}, v_{d}) over a predetermined time range is determined for the scalar product associated with the same weight vector (gₐ, g_{b}, g_{c}, g_{d}) in each case and said variance is associated with the weight vector,
- **in that** the individual variances (vₐ, v_{b}, v_{c}, v_{d}) and, optionally, a further, predetermined summand are weighted and added up with weight values (wₐ, w_{b}, w_{c}, w_{d}) of a further weight vector (w), and
- **in that** the sum thus obtained or a sequence of sums recorded immediately one after the other in this way of the classification analysis (105) is taken as the test value (T).

8. The method according to claim 7, **characterised in that** the weight vectors (gₐ, g_{b}, g_{c}, g_{d}) and the weight values (wₐ, w_{b}, w_{c}, w_{d}), as well as, optionally, the further summand (s), are adapted to the person (1) so that the measure (M) determined in the classification analysis (105) is maximized, in particular by iteratively adapting, proceeding from predetermined start values, the weight vectors (gₐ, g_{b}, g_{c}, g_{d}), the weight values (wₐ, w_{b}, w_{c}, w_{d}), and, optionally, the further summand (s) until the classification analysis (105), on the basis of the already determined test data, provides a maximum measure (M) for the distinguishability.

9. The method according to claim 7 or 8, **characterised in that** the EEG data are subjected channel by channel to bandpass filtering (201) before being evaluated by means of classification analysis, wherein the filtered signal (s) contains, in particular exclusively, frequencies between 8 Hz and 30 Hz.

10. The method according to one of the preceding claims, **characterised in that** the EEG data or the data on which the classification analysis (105) is based, in particular the results of an averaging, the evoked potentials derived from the EEG data or the EEG data after carrying out an event-related desynchronisation or the EEG data are displayed, preferably to the person (1) and/or an operator leading the method, or are evaluated with a statistical test and only significant changes are displayed.

11. The method according to one of the preceding claims, **characterised in that** the measure (M) for whether the EEG data associated with a certain stimulus (S) can be distinguished from the EEG data associated with a stimulus (S) of a different type is carried out by means of the following types of classification analysis:
- discriminant analysis, in particular linear discriminant analysis,
- support vector machines and
- neural networks.

12. The method according to one of the preceding claims, **characterised in that** the method is carried out on several, in particular successive, days, optionally repeatedly, in particular with the same stimuli (S), wherein the measure (M) for the perceptive faculty of the person (1) is determined separately for each day and the measure (M) which indicates the greatest perceptive faculty is used as a measure (M) for the perceptive faculty of the person (1).

## Revendications

1. Procédé pour quantifier la capacité de perception d'une personne (1) qui ne dispose pas d'autres moyens de communication, tels que, notamment, la parole ou les gestes et pour communiquer avec cette personne (1) ultérieurement,
a) dans lequel un ensemble d'au moins deux types de stimuli (S) différemment perceptibles possibles, qui peuvent être appliqués aux personnes testées (1) est prédéterminé, dans lequel des sollicitations vibratoires sont appliquées à différentes parties du corps et/ou avec une intensité et/ou une durée différentes à la personne (1) au moyen d'unités vibratoires et
b) la personne testée (1) se voit confier des tâches mentales à exécuter en présence d'un stimulus (S) en fonction du type de stimulus (S),
c) dans lequel une pluralité d'étapes de test sont effectuées, dans lequel pour chacune des étapes de test, respectivement
- un type de stimulus (S) est sélectionné parmi l'ensemble des types de stimuli (S) possibles, notamment selon des critères aléatoires,
- un stimulus (S) du type sélectionné respectif est appliqué à la personne (1),
- les données EEG de la personne sont déterminées et enregistrées dans un intervalle de temps avant, pendant ou après l'application du stimulus (S) respectif, l'intervalle de temps ayant de préférence une durée de 1 à 10 secondes et
- on fait la moyenne des données EEG respectivement déterminées et les potentiels évoqués ainsi déterminés sont affectés au stimulus (S) respectif,
d) une mesure (M) est déterminée au moyen d'une analyse de classification (105), pour savoir si les données EEG affectées à un stimulus (S) particulier peuvent être distinguées des données EEG affectées à un stimulus (S) de type différent et
e) dans lequel la mesure (M) pour la différenciation des données EEG de différents stimuli (S) est utilisée comme mesure pour la capacité de perception, indiquant dans quelle mesure elle est encore capable de communiquer avec son environnement,
- dans lequel, après que la mesure (M) de la capacité de perception de la personne (1) a été déterminée, il est ordonné d'effectuer les activités mentales précédemment utilisées pour la communication, en particulier pour répondre de manière positive ou négative à des questions,
- dans lequel la personne (1) exerce des activités mentales en réponse à la question posée,
- dans lequel les données EEG de la personne (1) sont déterminées et enregistrées dans des intervalles de temps prédéterminés pendant ou après la question,
- dans lequel les données EEG enregistrées respectives sont classées au moyen de l'analyse de classification (105) précédemment effectuée et
- dans lequel les résultats (E) respectifs déterminés de la classification sont utilisés comme contenu de la communication et, si nécessaire, sont tenus à disposition.

2. Procédé selon la revendication 1, **caractérisé en ce que** la mesure (M) est déterminée en examinant la probabilité avec laquelle l'application de l'analyse de classification (105) aux données EEG individuelles associées aux types de stimuli indique le stimulus correct dans chaque cas.

3. Procédé selon l'une des revendications 1 ou 2, **caractérisé**
- **en ce que** l'ensemble des types de stimuli (S) est prédéterminé par des tonalités différentes, en particulier avec une durée, une fréquence et un volume différents, dans des fréquences audibles par un être humain et la tonalité respective est jouée à la personne (1).

4. Procédé selon l'une des revendications précédentes, **caractérisé en ce que** l'ensemble des types de stimuli (S) comprend des stimulations visuelles pour un ou les deux yeux et/ou d'intensité et/ou de durée différentes appliqués à la personne (1) au moyen d'un écran ou au moyen de moyens d'éclairage.

5. Procédé selon l'une des revendications précédentes, **caractérisé en ce que** l'ensemble de types de stimuli (S) comprend des stimulations électriques sur différentes parties du corps et/ou d'intensité et/ou de durée différentes appliqués à la personne (1) au moyen de stimulateurs électriques.

6. Procédé selon l'une des revendications précédentes, **caractérisé en ce que** la personne (1) procède à l'une des activités mentales suivantes en fonction du type de stimulus (S) respectif, en particulier en présence d'une stimulation tactile par une unité vibratoire dans une région corporelle prédéterminée :
- compter ou calculer,
- penser aux mouvements des parties du corps, en particulier des extrémités de la moitié droite ou gauche du corps, de préférence les bras ou les mains.

7. Procédé selon l'une des revendications précédentes, **caractérisé en ce qu'**une évaluation des données EEG enregistrées est effectuée en combinant les données EEG individuelles enregistrées en même temps, des différents canaux EEG en un vecteur de signal (s) respectif,
- **en ce qu'**un nombre, en particulier quatre, de vecteurs de poids (gₐ, g_{b}, g_{c}, g_{d}) est prédéterminé, qui ont le même nombre d'éléments que les vecteurs de signal (s),
- **en ce que**, pour chaque instant, le produit scalaire (p_{a,} p_{b}, p_{c}, p_{d}) du vecteur de signal (s) déterminé avec chacun des vecteurs de poids (gₐ, g_{b}, g_{c}, g_{d}) est calculé et les produits scalaires (p_{a,} p_{b}, p_{c}, p_{d}) calculés respectifs sont affectés au vecteur de poids respectif (gₐ, g_{b}, g_{c}, g_{d}),
- **en ce que** la variance (vₐ, v_{b}, v_{c}, v_{d}) est déterminée dans chaque cas sur une plage de temps prédéterminée parmi les produits scalaires affectés au même vecteur de poids (gₐ, g_{b}, g_{c}, g_{d}) et est affectée au vecteur de poids,
- **en ce que** les variances individuelles (vₐ, v_{b}, v_{c}, v_{d}) et, le cas échéant, un autre additionneur prédéfini sont pondérés et additionnés aux valeurs de poids (wₐ, w_{b}, w_{c}, w_{d}) d'un autre vecteur de poids (w) et
- **en ce que** la somme ainsi obtenue ou une suite de sommes ainsi enregistrées immédiatement l'une après l'autre de l'analyse de classification (105) est utilisée comme valeur d'essai (T).

8. Procédé selon la revendication 7, **caractérisé en ce que** les vecteurs de poids (gₐ, g_{b}, g_{c}, g_{d}) et les valeurs de poids (wₐ, w_{b}, w_{c}, w_{d}), ainsi que éventuellement l'autre additionneur (s), sont adaptés à la personne concernée (1) de sorte que la mesure (M) déterminée dans l'analyse de classification (105) soit optimisée, en particulier en adaptant de manière itérative, à partir de valeurs de départ prédéterminées, les vecteurs de poids (gₐ, g_{b}, g_{c}, g_{d}), les valeurs de poids (wₐ, w_{b}, w_{c}, w_{d}) et, le cas échéant, l'autre additionneur (s) jusqu'à ce que l'analyse de classification (105), basée sur les données de test déjà déterminées, fournisse une mesure maximale (M) pour la différenciation.

9. Procédé selon la revendication 7 ou 8, **caractérisé en ce que** les données EEG sont soumises canal par canal à un filtrage passe-bande (201) avant d'être évaluées par analyse de classification, le signal (s) filtré contenant notamment exclusivement des fréquences entre 8 Hz et 30 Hz.

10. Procédé selon l'une des revendications précédentes, **caractérisé en ce que** les données EEG ou les données sur lesquelles se base l'analyse de classification (105), en particulier les résultats d'une moyenne, les potentiels évoqués dérivés des données EEG ou les données EEG après l'exécution d'une désynchronisation liée à un événement ou les données EEG, de préférence de la personne (1) et/ou de l'opérateur qui dirige le procédé, sont affichés ou évalués avec un test statistique et seules les modifications importantes sont affichées.

11. Procédé selon l'une des revendications précédentes, **caractérisé en ce que** la mesure (M) de la possibilité de différencier les données EEG associées à un stimulus particulier (S) des données EEG associées à un autre type de stimulus (S) est effectuée au moyen d'un des types suivants d'analyse de classification :
- analyse discriminante, en particulier analyse discriminante linéaire,
- machines à vecteurs de support,
- réseaux neuronaux.

12. Procédé selon l'une des revendications précédentes, **caractérisé en ce que** le procédé est réalisé sur plusieurs jours, notamment successifs, éventuellement plusieurs fois, notamment avec les mêmes stimuli (S), la mesure (M) de la capacité de perception de la personne (1) étant déterminée séparément pour chaque jour et la mesure (M) qui indique la capacité de perception maximale étant utilisée comme mesure (M) de capacité de perception de la personne (1).
